(19) **Europäisches Patentamt / European Patent Office / Office européen des brevets**

(11) **EP 1 165 149 B1**

(12) **EUROPEAN PATENT SPECIFICATION**

(45) Date of publication and mention
of the grant of the patent:
**13.04.2011 Bulletin 2011/15**

(21) Application number: **01901112.1**

(22) Date of filing: **22.01.2001**

(51) Int Cl.:
***A61L 2/06*** *(2006.01)*   ***A61L 2/24*** *(2006.01)*

(86) International application number:
**PCT/CZ2001/000002**

(87) International publication number:
**WO 2001/058498 (16.08.2001 Gazette 2001/33)**

(54) **REGULATION OF STEAM SUPPLY IN STERILIZER**

DAMPFZUFUHRREGULIERUNG IN STERILISATOREN

REGULATION DE L'ALIMENTATION EN VAPEUR D'UN STERILISATEUR

(84) Designated Contracting States:
**AT BE CH CY DE DK ES FI FR GB GR IE IT LI LU MC NL PT SE TR**

(30) Priority: **28.01.2000 CZ 20000323**

(43) Date of publication of application:
**02.01.2002 Bulletin 2002/01**

(73) Proprietor: **BMT Medical Technology s.r.o.
656 60 Brno (CZ)**

(72) Inventors:
• **HABROVEC, Michal
616 00 Brno (CZ)**

• **HODAN, Ivan
612 00 Brno (CZ)**

(74) Representative: **Kendereski, Dusan
Lidicka 51
602 00 Brno (CZ)**

(56) References cited:
**EP-A- 0 015 328     WO-A-90/06779
US-A- 4 164 538     US-A- 4 238 447
US-A- 4 284 600**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Technical Field

The invention relates to the method intended for steam supply into a steam sterilizer chamber and equipment intended for realization of said method.

## State of Art

Steam supply into a sterilizing chamber of large steam sterilizers has been solved usually so that steam is first supplied into a jacket of the sterilizing chamber of the sterilizer that is thus pre-heated; and then said steam is supplied through a feeding valve into the sterilizing chamber itself

Document US 4164538 discloses a load conditioning control method for steam sterilization in a double walled chamber structure aimed in particular to deaerating of sterilization chamber and the charge and evaluation of the charge characteristics. The method operates during the preparatory conditioning phase that precedes the sterilization itself. During said conditioning phase the chamber and the materials therein are subject to a plurality of cyclic pressure pulses consisting in removing air (evacuating phase) and heating (vapor injecting phase). The pressure sensor senses continuously chamber pressure in order to evaluate the characteristics of the charge by the means of detection of speed of increase or decrease of chamber pressure during the conditioning phase. The pressure regulator is designed only to provide the constant pressure high enough to enable the flow of steam into the sterilization chamber thanks to existence of pressure gradient. Flow of steam during pressurization (and also during evacuation) of the chamber is controlled only by opening or closing of the valve. At the conclusion of the conditioning pulsing, the chamber is pressurized with steam to a level producing the desired sterilization temperature, and maintaining at this temperature for a predetermined time (column 6, line 63-68). Since the pressure in the jacket is constant and high enough in order to ensure the pressure gradient during the whole pressurization the jacket is in this case pre-heated and the temperature of the jacket is much higher than the desired sterilization temperature. This high temperature in the jacket results in more intensive heat transfer into the chamber by a spurious way that introduces the disturbance variable into the regulation process in the chamber.

A jacket control for steam sterilizers is described in WO 90/06779. A steam sterilizer having an improved jacket control comprises a steam chamber for receiving materials to be sterilized. A steam jacket substantially surrounds the steam chamber. Means are provided for admitting steam into the jacket. A chamber valve is connected between the steam jacket and the chamber for controllably admitting steam into the chamber from the jacket. A control circuit controls the operation of the means for admitting steam and the chamber valve such that the pressure in the steam chamber follows the pressure in the steam jacket. Another steam sterilizing process is disclosed in US 4238447. A process for sterilizing laboratory and hospital glassware, liquids, instruments, garments and the like comprises the steps of inserting the materials to be sterilized in a closed, sealed, jacketed sterilizer, introducing steam into the jacket of said sterilizer to preheat said jacket, interconnecting said jacket and the interior of said stabilizer initially to displace any air within said sterilizer, continuing to interconnect the jacket and the interior of said sterilizer; providing the jacket with steam at one of two different predetermined pressures; sensing the temperature within said sterilizer and introducing steam into said jacket at said higher pressure in response to said temperature sensitive means to maintain a variable, predetermined temperature within said sterilizer. Above mentioned methods do not operate with the value of the pressure gradient between the jacket and the chamber and therefore are not able to meet strong requirements laid on the sterilization process as described below.

Jacket pre-heating is a desirable event but it has also certain practical limitations. It is desirable to maintain the area having approximately the same temperature both round the sterilizing chamber and in the chamber itself in order to ensure thermal isolation of the chamber from its environment. This has the positive impact both to temperature time behaviour in the chamber (temperature fluctuations) and to temperature distribution in the chamber and thus also in the material sterilized. The pre-heated jacket also contributes significantly to good drying effect of the steam sterilizer. High demands have been made nowadays to quality of the above-mentioned parameters. On the other hand, too high temperature in the jacket results in more intensive heat transfer into the chamber by a spurious way that introduces the disturbance variable into the regulation process in the chamber. The requirements demanding that temperatures measured in the sterilizer chamber shall be within the specified range (3 K) of the sterilizing temperatures during their maintaining shall be particularly kept. Moreover, said temperatures measured shall not vary by more than 1 K and shall not differ by more than 2 K in any point measured. The time interval necessary for reaching the sterilizing temperature in the coldest and warmest parts of the sterilizer chamber shall not exceed 15 seconds and 30 seconds for the sterilizer fitted with the chamber which volume is maximum 800 l and more, respectively. Steam is by-passed from the jacket to the sterilizer chamber usually continuously. In some cases, supply velocity is limited so that the feeding valve is closed and opened by turns while its parameters are set fixedly. In any case, this does not result in the linear pressure increase in the chamber because steam passes into the chamber considerably quicker at the higher pressure gradient in comparison with the lower one. Speed of pressure increase in the chamber depends considerably on the charge size, its thermal capacity and the level of heating-up of the sterilizing chamber itself. Proportional valves can be also used for steam passing-by into the chamber; nevertheless, they features with high price and increased failure rate.

Considering the requirements for parameters of the temperature distribution in the sterilizer chamber and the material sterilized, pressure in the jacket of the sterilizing chamber has been currently regulated in dependence on the instantaneous pressure value measured in the sterilizing chamber. Pressure in the jacket is usually maintained at the value that is higher only by the constant pressure difference, $\Delta p$, than the actual pressure in the sterilizing chamber. Steam source for the sterilizing chamber is thus affected by steam pressure at the site - at the target place - in the chamber itself. It should be emphasised that $\Delta p$ value itself represents the constant value present during steam passing-by in the currently known applications. Pressure difference deduced from the constant temperature difference of the saturated steam between the jacket and the sterilizing chamber is used in some cases. The temperature difference specified is converted automatically to the pressure difference, $\Delta p$, that corresponds to the actual temperature of the saturated water steam. So this is the case of the pressure difference, $\Delta p$, expressed by the constant temperature difference.

The disadvantage of the current state of art is the fact that it is rather problematic to keep the temperature profile in the empty sterilizing chamber of the steam sterilizer. Temperature in the chamber shall be measured simultaneously in many points in the chamber, essentially, in its whole volume. The temperature difference between the individual points measured at the beginning of the period when the sterilizing temperature is maintained does not usually comply with the requirements specified in the standard. It is also problematic to keep the demanded time interval between the values of the sterilizing temperature reached in the warmest and coldest parts of the chamber.

Temperature non-homogeneities and temperature increase in the phase when steam is supplied into the sterilizing chamber are mostly, according to the thermodynamics laws, caused by quick gas compression in the sterilizing chamber. The additional heating-up thus occurs in the chamber when the excessive heat cannot be transferred into chamber walls or into the material - the sterilized charge. Another disturbance variable thus arises for the regulation demands. Said variable features with its unpredictable character; it depends, among others, also on the volume and material of the objects sterilized that usually is not known in advance. The process that is able to influence the steam velocity supplied into the chamber can solve the above-mentioned problems.

But only the single limitation of the speed of pressure increase is not able to react on the different conditions of steam consumption depending on the different charge size and thermal capacity of the sterilizer because the levels of chamber pre-heating are different. The more intensive steam condensation and thus bigger off-take of the heat energy supplied occurs in the cold chamber in comparison with that one that has been still pre-heated due to the previous operation. The same is valid also for the full chamber, in contrast with the empty one.

This invention is aimed at creating such system for steam supply that can be capable to react to the actual conditions occurring in the sterilizing chamber of the steam sterilizer, charge size and its thermal capacity, and that further enables keeping of the parameters demanded not only for the full sterilizing chamber but also for the measurement of the temperature profile in the empty sterilizing chamber.

**Disclosure of Invention**

The disadvantages mentioned above can be rectified using the method for steam supply into the sterilizing chamber of the steam sterilizer according to claim 1.

For steam supply into the steam sterilizer chamber, it seems advantageous also to regulate the speed of pressure increase in the chamber by means of opening or closing the feeding valve supplying steam into the jacket based on the correspondence between the actual and demanded speed of steam supplied into the chamber while the valve is opened permanently.

For steam supply into the steam sterilizer chamber, it is advantageous when the speed of pressure increase in the sterilizing chamber of the steam sterilizer is regulated by opening and closing of the feeding valve supplying steam into the sterilizing chamber based on the correspondence of the actual and demanded speed of steam supplied into the chamber of the steam sterilizer.

It seems to be natural if controlling of pressure difference, $\Delta p$, between the jacket and the sterilizing chamber is performed in the starting phase A using an iterative step, within the interval of which the pressure difference, $\Delta p$, between the jacket and the sterilizing chamber increases, decreases or remains unchanged based on the correspondence of the actual and demanded speed of steam supplied into the chamber while the feeding valve supplying steam into the jacket reacts to the amount of the pressure difference, $\Delta p$, between the jacket and the sterilizing chamber independently on the iterative step.

It is also advantageous when speed of the pressure increase in the ending phase C is regulated using the iterative step during which the feeding valve supplying steam into the chamber is opened or closed based on the correspondence of the actual and demanded speed of steam supplied into the chamber.

The equipment intended for performing the method of steam supply into the sterilizing chamber of the steam sterilizer characterized in that it consists of the sterilizing chamber fitted with the jacket into which space both the outlet of the feeding valve supplying steam into the jacket and the input of the second pressure sensor and the inlet of the feeding valve supplying steam into the sterilizing chamber are introduced, while the outlet of the feeding valve of the sterilizing chamber and the input of the first pressure sensor are introduced into the chamber area; in addition, the initialisation block and the output of the first comparator which one input is connected to the constant memory and the second one is connected both to the derivative member and the input of the second comparator which output is connected to the second totalling member and the second input is connected to the constant memory block while the second totalling member is further connected with the input of the third comparator and the first totalling member, while the further input of the third comparator is connected with the constant memory block and its output with the feeding valve supplying steam into the jacket while the derivative member is further connected both to the first pressure sensor and the first totalling member that is further connected to the second pressure sensor, are connected to the feeding valve.

It is advantageous when the equipment intended for performing the method of steam supply into the sterilizing chamber of the steam sterilizer is controlled by the block of microprocessor automatics characterized in that it consists of the sterilizing chamber with the jacket in which space both the outlet of the first feeding valve supplying steam into the jacket and the input of the second pressure sensor and the input of the second feeding valve of the sterilizing chamber are introduced while the outlet of the second feeding valve of the sterilizing chamber and the input of the first pressure sensor are introduced into the chamber space while the microprocessor automatics block is connected to the second feeding valve, and both the feeding valve and the second pressure sensor and the first pressure sensor are further connected to the block of the microprocessor automatics.

The advantage of controlling the steam supply into the chamber of the steam sterilizer and the equipment for performing said method according to the invention is that speed of steam supply into the sterilizing chamber will be sufficient to comply with the practical requirement demanding the shortest supply time that is simultaneously low enough to avoid temperature overshoot caused by gas compression in the chamber, and also in the case when the chamber is empty or contains only small charge.

It is advantageous that the temperature in the individual spots of the sterilizing chamber will be within the demanded range during the most critical heating-up phase, i.e., at the beginning of the sterilization cycle.

It is also advantageous that the time interval between the moment when the sterilizing temperature is reached in the coldest and warmest spot of the chamber will not be longer than the demanded one.

## List of sketches given in the drawings

The invention will be described by means of the following sketches: Fig. 1 illustrates the layout of the sterilizing chamber of the sterilizers with the regulating elements; Fig. 2 illustrates the diagrams showing the phase process of steam supply into the sterilizing chamber; Fig. 3 illustrates the iterative step in the starting phase A when pressure is changed by the value demanded still before the demanded time interval elapsed while the rest of the time interval is lower than the dead band value; Fig 4 illustrates the iterative step in the starting phase A when pressure is changed by the demanded value still before the demanded time interval elapsed while the rest of the time interval is within the dead band value; Fig. 5 illustrates the iterative step in the starting phase A when the demanded time interval elapsed earlier than pressure was changed by the demanded value; Fig. 6 illustrates the iterative step in the ending phase C; Fig. 7 illustrates the layout of the sterilizing chamber of the sterilizer fitted with the microprocessor automatics.

## Description of exemplary performance

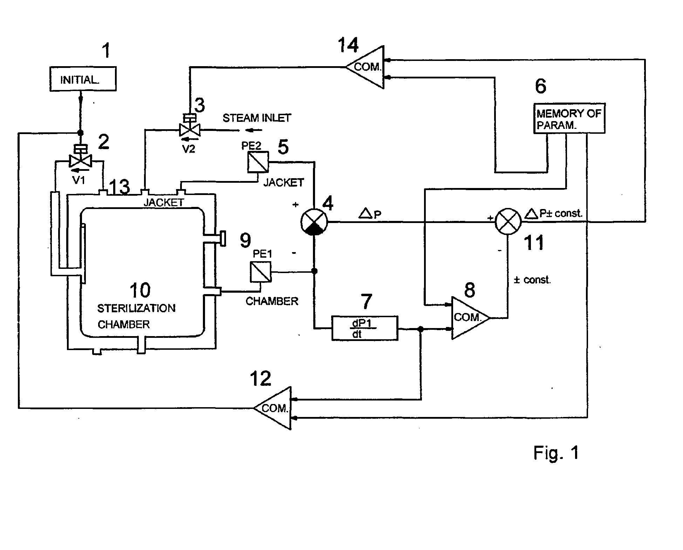

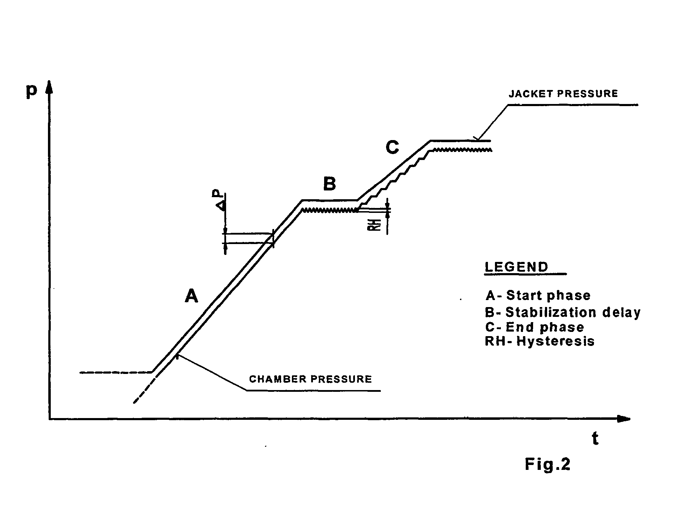

The method for the regulation of steam supply into the sterilizing chamber of the steam sterilizer will be described using one of the possible examples showing how the method can be performed. The general scheme of the equipment intended for the method performance is described in Fig. 1. The method is performed using the speed regulation of pressure increase in the sterilizing chamber. The pressure increase is approximately linear. The whole process of steam supply and thus heating-up of the chamber of the steam sterilizer in order to reach the working temperature can be advantageously divided into two active phases, $\underline{A}$ and $\underline{C}$, between which the stabilization dwell phase $\underline{\mathbf{B}}$ is included. The above-mentioned individual phases are illustrated in Fig. 2.

The indirect regulation is used during the starting phase $\underline{\mathbf{A}}$ of steam supply into the sterilizing chamber using the method of affecting $\Delta p$ value. According to the instantaneous speed of pressure increase, the pressure difference, $\Delta p$, between the jacket and the sterilizing chamber is increased or decreased. If the speed of pressure increase in the sterilizing chamber is low, $\Delta p$ is increased using the more intensive steam supply into the jacket from the primary steam source. Thus the temperature gradient between the jacket and the sterilizing chamber is increased and steam attempts to pass into the sterilizing chamber. On the contrary, if the speed of pressure increase in the sterilizing chamber is high, $\Delta p$ is decreased because the steam supply from the source into the chamber is lower. The lower pressure gradient results then in the lower steam supply into the sterilizing chamber and thus the speed of pressure supply into the sterilizing chamber is decreased. The demanded speed for steam supply into the sterilizing chamber is specified by the parameters that can be set accordingly. The parameters are as follows: time and pressure level of supply element, so-called the partial level of the iterative step that represents one of many levels forming the whole supply phase. The whole starting phase **A** of steam supply into the sterilizing chamber results in one essential fact, namely, that - at the end of the starting phase **A** - $\Delta p$ is set to the value that is optimum for the given speed and load of the sterilizing chamber. $\Delta p$ value set as above represents the significant factor that also takes part into the further, i.e. ending phase **C**, of filling. The demand to minimise $\Delta p$ is caused by the effort to reach the lowest possible parasitical heat transfer from the jacket into the sterilizing chamber. Lets suppose that the most adverse conditions for ensuring the sufficient heat profile are present in the sterilizing chamber of the sterilizer. Such conditions are present, for example, in the empty sterilizing chamber. The parameters that are still sufficient but simultaneously do not slow down steam supply can be found when the parameters (time and pressure level, i.e., speed) of the supplying element, so-called the partial level of the iterative step, are step by step changed and the heat profile of the sterilizing temperature is measured using the above-mentioned parameters. The demand for the possible shortest duration of starting up of the sterilizing cycle is significant. When the above-mentioned method of the regulation is applied, the parameters for the full sterilizing chamber will not be affected. Simultaneously, $\Delta p$ value set automatically to the somewhat higher value during the phase keeps the sufficient speed of steam supply. Said speed will be thus always the same for the different charges present in the sterilizing chamber and for various pre-heating levels and so it will correspond to the demanded value. This is, of course, valid in the case that $\Delta p$ value does not reach the upper or the lower limits and also in the case that the speed is selected within the range of the given technical layout (cross-sections of pipelines and valves, steam source, etc.). The value exceeding the upper limit value will result in the parasitic heating-up in the sterilizing chamber because the jacket round the sterilizing chamber will be over-heated; on the contrary - the value below the lower limit will stop steam flow completely. Due to the starting phase **A** (filling) the sterilizing chamber is heated-up closely below the sterilizing temperature.

The stabilizing dwell **B**, see Fig. 2, that shall balance the temperature field in the sterilizing chamber before the ending phase C (steam supply) is started follows the starting phase **A**.

In the ending phase **C** (about 4°C below the sterilizing temperature value), the direct regulation of speed of pressure increase in the sterilizing chamber is used when the feeding valve between the jacket and the sterilizing chamber is controlled and $\Delta p$ value is set to its optimum and has not been already changed. Similarly to the starting phase **A** when steam is supplied, the feeding speed is also given by the time and the pressure level of the feeding element, so-called the partial level of the iterative step, while said parameters are independent on those one used in the starting phase **A**. If the feeding speed is low, more steam is passed from the jacket; if it is higher than demanded, the feeding valve is closed sooner. The ending phase **C** enables optimisation of the transient process during the transition to the sterilizing exposure. The ending phase **C** is completed at the moment when pressure in the jacket is equal to that one corresponding with the demanded sterilizing temperature. At this moment, $\Delta p$ function is interrupted, pressure in the jacket is regulated to the pre-specified level and the sterilizer waits until the temperature in the chamber reaches the level specifying the beginning of the sterilization cycle.

One of the advantageous performances of the equipment intended for the method according to this invention illustrates the layout of the sterilizer chamber fitted with the regulation elements, see Fig. 1. According to this equipment layout, the sterilizing chamber **10** is fitted with the jacket **13** while both parts forms one unit that cannot be dismantled. Both the outlet of the first feeding valve **3** supplying steam into the jacket and the input of the second pressure sensor **5** and the inlet of the second feeding valve **2** of the sterilizing chamber **10** are introduced into the jacket space 13 while the outlet of the second feeding valve **2** of the sterilizing chamber **10** and the input of the first pressure sensor **9** are introduced into the space of the sterilizing chamber **10** while the initialising block **1** and the output of the first comparator **12**, which one input is connected to the constant memory block **6** and its second input is connected both to the derivative member **7** and to the input of the second comparator **8** which one output is connected to the second totalling member **11** and the second input is connected to the constant memory block **6**, are introduced into the second feeding valve **2**. The second totalling member **8** is further connected with the input of the third comparator **11** and the first totalling member **4** while the further input of the third comparator **11** is connected with the constant memory block **6** and its output is connected with the first feeding valve **3** supplying steam into the jacket **13** while the derivative member **7** is further connected both to the first pressure sensor **9** and the first totalling member **4** that is further connected to the second pressure sensor **5**.

Handling with the equipment is clear from Fig. 1 through Fig. 6. The process of steam supply into the chamber starts when the starting phase **A** begins, see Fig. 2. In this moment, the initialising block **1** opens the second feeding valve **2** intended for steam passing-by from the jacket **13** into the sterilizing chamber **10** of the steam sterilizer. The automatic elements begin to monitor the supply speed. The initialising block **1** does not affect the process any more. The first comparator **12** is not also involved in the beginning phase **A**. Pressure in the sterilizing chamber **10** and pressure in the jacket is converted to the electrical value by means of the first pressure sensor **9** and the second pressure sensor **5**, respectively. Signals of both pressure values are transferred to the totalling member **4**; the pressure value obtained from the chamber is considered as negative. Both values are summarized in the member **4** and their result is the actual value, $\Delta p$. The signal indicating the pressure in the chamber is converted in the derivative member **7** to the speed of the pressure increase in the sterilizing chamber **10**. The demanded speed value is stored in the constant memory block **6**. The second comparator **8** generates the constant that is based on the actual and demanded speed of the steam supplied into the sterilizing chamber **10**. The resulting constant is either positive or negative or it can be also zero. In the second totalling member **11**, the constant mentioned is summed with the actual value, $\Delta p$. The result is the value $\Delta p \pm$ constant that represents in fact the value of $\Delta p$ for the further iterative step and considers if the speed of steam supply into the sterilizing chamber **10** should be increased (the positive constant) or decreased (the negative constant) or let unchanged (the zero constant). This is the principle of the whole regulation process in the starting phase **A** when steam is supplied into the sterilizing chamber **10**. Certain dead band is included into the system that avoids system pulsation; if said band is reached, the constant on the output of the second totalling member **11** equals zero. The value of $\Delta p \pm$ constant shall be limited by the limit values from the practical point of view. This requirement is realized in the third comparator **14** into which the limiting parameters are transferred from the constant memory block 6. The acting value that controls the first feeding valve 3 is already available on the output of the third comparator **14**. The first feeding valve **3** supplied steam from the steam source into the jacket **13**. The source is not illustrated in the scheme; either the self-contained steam generator or the external distribution system of saturated steam can be used. At the end of the starting phase **A**, the third comparator **14** stores the last output value and does not consider further changes present in the inputs. $\Delta p$ has not been already regulated but its last value participates actively in the further heating-up of the sterilizing chamber **10**. All other blocks that were in operation up to now continue in their operation.

After the starting phase **A** is completed, the stabilizing dwell phase **B** follows, see Fig. 2; it is intended for balancing of the temperature field in the sterilizing chamber **10** before the ending phase **C** (steam supply) begins. Here the second feeding valve **2** is regulated from the output of the first comparator **12** using the single on/off method and the certain regulating hysteresis. The speed of pressure increase in the chamber **10** from the derivative member **7** and the zero reference value from the constant memory block **6** are transferred into said first comparator **12**. During the dwell, the temperature values measured in the different points of the sterilizing chamber **10** get closer to each other.

Then the ending phase **C**, steam supply into the sterilizing chamber **10**, follows, see Fig. 2. The demanded speed of steam supply is generated again in the constant memory block **6** and the first comparator **12** controls the second feeding valve **2** by comparing of both values. The second feeding valve **2** is opened or closed depending on the actual demand, i.e. if the speed of steam supply needs to be increased, decreased or kept. $\Delta p$ remains constant, namely, the same that was set at the end of the starting phase **A** of steam supply, i.e. set optimally.

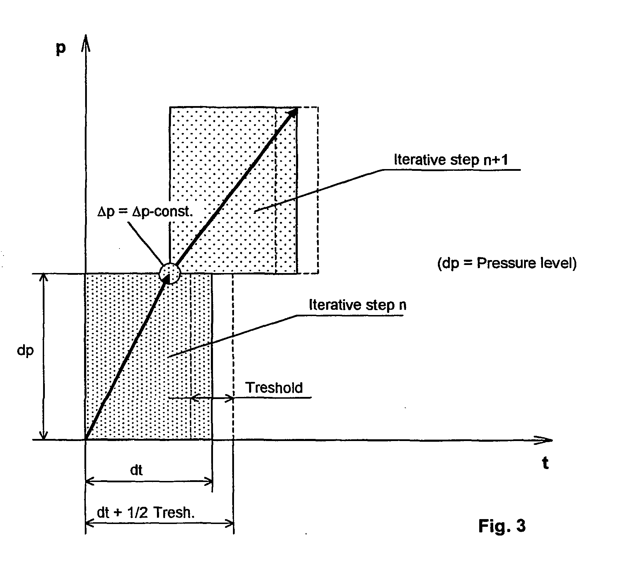

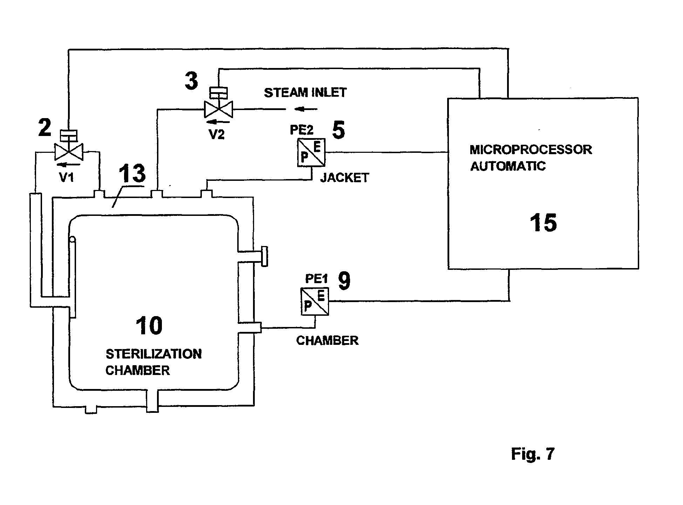

As it is illustrated in Fig. 7, the whole equipment can be advantageously controlled by the block **15** fitted with the microprocessor automatics and most of the actions described above are provided by software. The important operations are performed by timers that interrupt the main program at the regular intervals in order to perform some action. After the action is completed, they return the program control precisely into the place where the main program was interrupted. The timing devices enable continuous sampling of pressure or speed (their changes in time) and compare the values obtained with the decisive values - both the constants and variables. The iterative step is important for monitoring of pressure increase in the sterilizing chamber **10**. It is the time interval during which the decrease of the time element from the set value to zero value (time decrementation) is monitored together with the pressure level, i.e. the distance by which pressure is increased per the time element. The iterative step corresponds with the selectable parameters for steam supply, as described above. At the end of each iterative step, the action is performed that affects the development for the next step, e.g. $\Delta p$ is changed, the valve is opened, etc. Both the starting phase **A** and the ending phase **C** - steam supply into the sterilizing chamber **10** - are performed by the iterative step method. The iterations steps are of course different for each phase. Their algorithm performed by the relevant software is described below. The graphical flow chart of the iteration steps for the starting phase A is described in Fig. 3, 4 and 5.

The iteration step in the starting phase **A**. The moment when pressure in the sterilizing chamber **10** is changed by dp value from the value obtained at the end of the previous iteration step and the elapsing time of said iteration step are simultaneously monitored by the software. Two different situations resulting in ending of the iteration step can occur:

  *A) Pressure was changed by dp still before time dt + the half of the dead band elapsed.*
  The following actions will be performed in this case:

    a) Duration of the iteration step is measured; its value can be as follows:

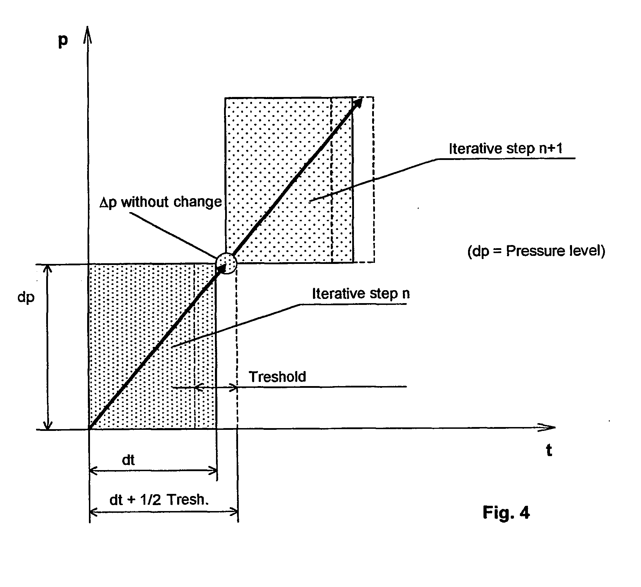

      1. It can be smaller than dt value decreased by the half of the dead band, see Fig. 3; in such case, the actual value $\Delta p$ is decreased by the constant decrease: $\Delta p = \Delta p$ - constant
      2. The value is within the dead band, see Fig. 4; $\Delta p$ remains unchanged.

b) The pressure for the next iteration step is set to the value of the actual pressure increased by dp.

c) Time for the next iteration step is set to dt + the half of the dead band.

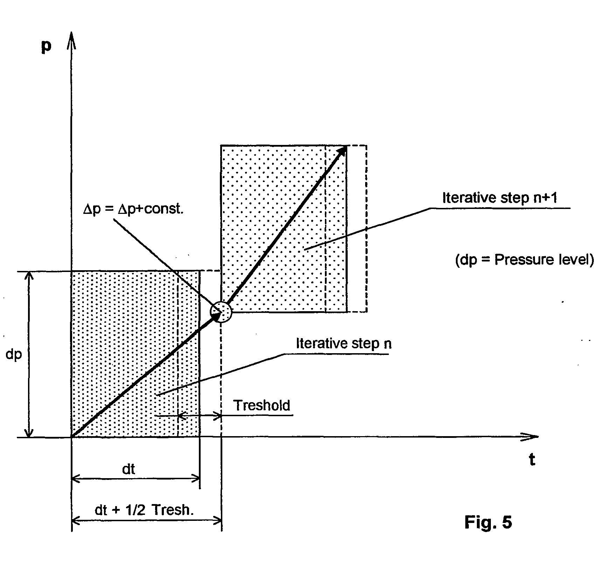

B) *Time dt + the half of the dead band elapses sooner that the pressure value is changed by dp.* See Fig. 5

The following actions will be performed in this case:

a) The actual value $\Delta p$ is increased by the constant increment:

$$\Delta p = \Delta p + \text{constant}$$

b) The pressure value for the next iteration step is set to the actual pressure value increased by dp.

c) Duration of the next iteration step is set to dt + the half of the dead band.

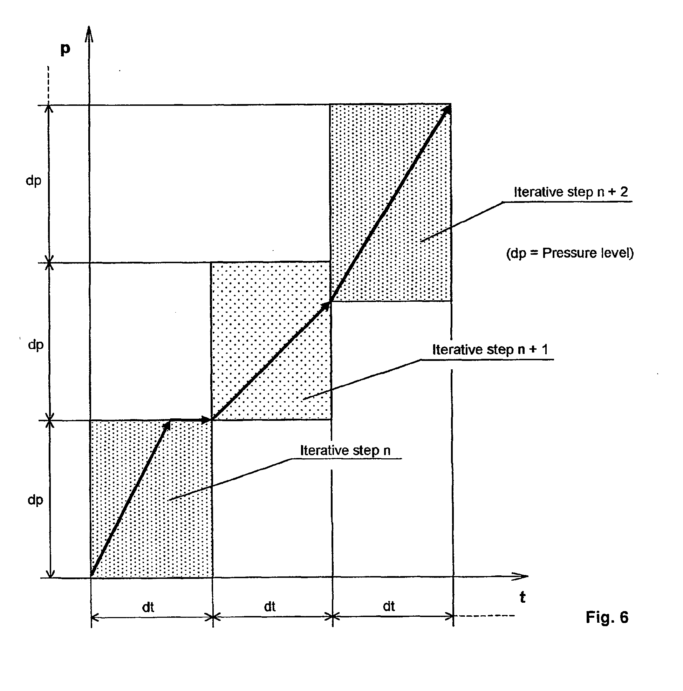

The graphical flow chart of the iteration steps for the ending phase **C** is illustrated in Fig. 6.

The iteration step in the ending phase C. The moment when pressure in the sterilizing chamber **10** is changed by dp value from the value obtained at the end of the previous iteration step and the elapsing time of said iteration step are simultaneously monitored by the software. In this case, the only possible way showing how the iteration step will be ended is that the time interval dt will elapse. Should the pressure value be changed sooner by dp value, steam supply into the sterilizing chamber **10** is interrupted and the equipment waits until dt value elapses. The following actions are performed at the end of the iteration step:

a) The pressure value for the next iteration step is set to the value of the previous iteration step increased by dp value.

b) Time for the next iteration step is set to dt value.

c) Steam from the jacket **13** is supplied into the sterilizing chamber **10**.

**Industrial applicability**

Method for regulation of steam supply into steam sterilizer chamber and equipment for realization of said method according to this invention can be applied for all types of steam sterilizers fitted with the chamber with the jacket.

**Claims**

1. The method for steam supply into the sterilizing chamber of the steam sterilizer performed in phases using the regulation of speed of steam pressure increase in the sterilizing chamber of the steam sterilizer by the regulation of the difference in the pressure values $\Delta p$ between the jacket and the sterilizing chamber **characterized In that** during the starting phase **A** of steam supply into the sterilizing chamber the pressure difference, $\Delta p$, between the jacket and the sterilizing chamber is increased or decreased according to the instantaneous speed of pressure increase in the sterilizing chamber and in the ending phase **C** the direct regulation of speed of pressure increase in the sterilizing chamber is used when a feeding valve between the jacket and the sterilizing chamber is controlled and $\Delta p$ value is set to its optimum and has not been already changed.

2. The method for steam supply into the sterilizing chamber of the steam sterilizer according to Claim 1 **characterized In that** the starting phase **A** is followed by a dwell phase **B** that precedes the ending phase **C**.

**Patentansprüche**

1. Verfahren zur Einspeisung von Dampf in die Sterilisationskammer des Dampfsterilisators, durchgeführt in einzelnen Phasen durch die Regelung der Geschwindigkeit des Druckanstiegs von Dampf in der Sterilisationskammer des Dampfsterilisators mittels der Ansteuerung der Druckdifferenz $\Delta p$ zwischen dem Mantel und der Sterilisationskammer, **dadurch gekennzeichnet, dass** während der Anfangsphase A der Einspeisung von Dampf in die Sterilisationskammer die Druckdifferenz $\Delta p$ zwischen dem Mantel und der Sterilisationskammer je nach der momentanen Geschwindigkeit des Druckanstiegs in der Sterilisationskammer erhöht oder erniedrigt wird und in der Schlussphase C die direkte Regelung der Geschwindigkeit des Druckanstiegs in der Sterilisationskammer durch die Ansteuerung des zwischen dem Mantel und der Sterilisationskammer angeordneten Einspeiseventils erfolgt, wenn der Wert von $\Delta p$ bereits optimal eingestellt und nicht veränderbar ist.

2. Verfahren zur Einspeisung von Dampf in die Sterilisationskammer des Dampfsterilisators nach Anspruch 1, **dadurch gekennzeichnet, dass** die Anfangsphase A von der Verweilphase B gefolgt wird, die wiederum der Schlussphase C vorhergeht.

**Revendications**

1. Le procédé pour le remplissage de la vapeur dans la chambre à stérilisation de stérilisateur à vapeur, pratiqué en phases par la conduite de la rapidité de rebond de tension de vapeur dans la chambre à stérilisation de stérilisateur à vapeur par la régulation de la différence des pressions $\Delta p$ entre l'enveloppe et la chambre à stérilisation, **caractérisé en ce qu'**à la phase initial A du remplissage de la vapeur dans la chambres à stérilisation, la différence des pressions $\Delta p$ entre l'enveloppe et la chambre à stérilisation est augmenté ou diminué d'après la rapidité immédiate du rebond de tension dans la chambre à stérilisation et à la phase finale C on appliquera la conduite directe de la rapidité du rebond de tension dans la chambre à stérilisation par le contrôle du robinete de remplissage entre l'enveloppe et la chambre à stérilisation, alors que la valeur de $\Delta p$ est déjà invariable, étant ajusté le plus afficacement.

2. Le procédé pour le remplissage de la vapeur dans la chambre à stérilisation de stérilisateur à vapeur **caractérisé en ce que** la phase initial A est suivie de la phase de pédale B, qui précéde la phase C finale.

Fig. 1

Fig.2

p

Iterative step n+1

Δp = Δp-const.

(dp = Pressure level)

Iterative step n

dp

Treshold

dt

dt + 1/2 Tresh.

**Fig. 3**

11

Fig. 4

Fig. 5

Fig. 6

**Fig. 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4164538 A
- WO 9006779 A

- US 4238447 A